# EUROPEAN PATENT APPLICATION

(11) **EP 4 717 230 A1**
(43) Date of publication of application: **01.04.2026**
(21) Application number: 25204629.7
(22) Date of filing: 25.09.2025
(51) Int. Cl.: A61F 2/00, A61F 2/26

(54) **IMPLANTABLE SYSTEM FOR TREATMENT OF CLIMACTURIA**

(30) Priority: 27.09.2024 US 202463699820 P
(71) Applicant: Coloplast A/S, 3050 Humlebaek (DK)
(72) Inventor: SHOUMAN, Khaled Said, Minneapolis, MN 55415 (US)

(57) **Abstract**

An implantable system for treatment of climacturia has an inflatable penile implant and an inflatable cuff that is implantable around at least a portion of a urethra. A pump is operatively coupled to the penile implant and to the cuff to inflate the penile implant in treating erectile dysfunction and to inflate the cuff in treating climacturia.

## Description

### Background

An implanted penile prosthesis is a proven approach to relieve erectile dysfunction for male users.

Some men suffer from a condition known as climacturia, which is also known as orgasm-associated incontinence, where a man leaks urine during ejaculation. Men who have had their prostate gland removed are at an increased risk of climacturia.

Physicians and patients would welcome a solution to treat climacturia.

### Summary

One aspect provides an implantable system for treatment of climacturia comprising:
a reservoir configured to contain a liquid;
a penile implant that is implantable in a penis and inflatable with the liquid contained in the reservoir, where the penile implant, when inflated, provides the penis with an erection in treatment of erectile dysfunction;
a cuff that is implantable around at least a portion of a urethra and inflatable with the liquid contained in the reservoir, where the cuff, when inflated, provides a coaptation of the urethra in treatment of climacturia; and
a pump comprising a pump bulb connected to a pump body, the pump bulb operable to move the liquid contained in the reservoir into the penile implant and into the cuff, the pump comprising:
   an inlet valve assembly comprising an inlet valve disposed in an inlet channel of the pump body and operable to allow a portion of the liquid contained in the reservoir to pass through the inlet channel and into the pump bulb,
   an exhaust valve assembly comprising an exhaust valve disposed in a first exhaust channel of the pump body and operable to allow a first portion of the liquid in the pump bulb to pass through the first exhaust channel and into the penile implant,
   a cuff valve assembly comprising a cuff valve disposed in a second exhaust channel of the pump body and operable to allow a second portion of the liquid in the pump bulb to pass through the second exhaust channel and into the cuff;
wherein operation of the pump bulb displaces the first portion of the liquid and the second portion of the liquid out of the pump bulb to inflate both the penile implant and the cuff.

An embodiment of the aspect above includes wherein operation of the pump bulb simultaneously inflates both the penile implant and the cuff.

An embodiment of the aspect above includes wherein the exhaust valve comprises a ball valve and the exhaust valve assembly further comprises a spring to bias the ball valve, and the exhaust valve assembly is configured to pressurize the penile implant in a range from 5 pounds-per-square inch (psi) to 20 psi, which in cgs units is in a range from 34.5 kPa to 68.9 kPa.

An embodiment of the aspect above includes wherein the cuff valve comprises a cuff ball valve and the cuff valve assembly further comprises a spring to bias the cuff ball valve, and the cuff valve assembly is configured to pressurize the cuff in a range from 0.5 pounds-per-square inch (psi) to 1.5 psi, which in cgs units is in a range from 3.5 kPa to 10.3 kPa.

An embodiment of the aspect above includes wherein the exhaust valve assembly further comprises an exhaust spring biasing an exhaust ball valve onto an exhaust valve seat, and the cuff valve assembly further comprises a cuff spring biasing a cuff ball valve onto a cuff valve seat;
wherein the exhaust valve assembly is configured to pressurize the penile implant in a range from 5 pounds-per-square inch (psi) to 20 psi, which in cgs units is in a range from 34.5 kPa to 68.9 kPa;
wherein the cuff spring has a spring constant selected to deflect to allow the cuff ball valve to close the second exhaust channel to prevent pressuring the cuff above 1.5 psi (10.3 kPa).

An embodiment of the aspect above includes wherein operation of the pump bulb simultaneously inflates both the penile implant and the cuff, and the exhaust valve assembly is operable to pressurize the penile implant in a range from 5 pounds-per-square inch (psi) to 20 psi, which in cgs units is in a range from 34.5 kPa to 68.9 kPa, and the cuff valve assembly is operable to close the second exhaust channel of the pump body to prevent pressure in the cuff exceeding 3 psi, which in cgs units is 20.7 kPa.

An embodiment of the aspect above includes wherein the cuff valve assembly is configured to close the second exhaust channel of the pump body to prevent additional liquid exiting the pump bulb and entering the cuff when a pressure in the cuff is in a range from 0.5 pounds-per-square inch (psi) to 1.5 psi, which in cgs units is in a range from 3.5 kPa to 10.3 kPa.

An embodiment of the aspect above includes wherein the second exhaust channel of the pump body comprises a reinforcing sleeve, and the reinforcing sleeve provides a valve seat downstream of the cuff valve.

The embodiment of the aspect above, further comprises:
a deflation valve disposed in the pump body, with the second exhaust channel of the cuff valve assembly located between the pump bulb and the deflation valve;
wherein the deflation valve has an open deflate position that allows pressurized liquid in the penile implant and pressurized liquid in the cuff to flow back into the reservoir.

An embodiment of the aspect above wherein the deflation valve is disposed in the pump body transverse to the inlet valve assembly and the exhaust valve assembly.

An embodiment of the aspect above includes wherein the deflation valve is movable linearly and squeezing the pump body displaces the deflation valve transversely to the open deflate position to allow the pressurized liquid in the penile implant and pressurized liquid in the cuff to bypass the pump bulb and flow back into the reservoir.

### Brief Description of the Drawings

The accompanying drawings are included to provide a further understanding of embodiments and are incorporated in and constitute a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Fig. 1 is a perspective view of one embodiment of an implantable system for treatment of climacturia including a penile implant, an artificial urinary sphincter cuff, and a pump.
Fig. 2 is a perspective view of the pump illustrated in Fig. 1.
Fig. 3 is a cross-sectional view of the pump illustrated in Fig. 2.
Fig. 4 is a cross-sectional view of the pump illustrated in Fig. 2 showing an embodiment of an inlet valve assembly.
Fig. 5 is a cross-sectional view of the pump illustrated in Fig. 2 showing an embodiment of an exhaust valve assembly.
Fig. 6 is a cross-sectional view of the pump illustrated in Fig. 2 showing an embodiment of a cuff valve assembly.
Fig. 7 is a cross-sectional view of the pump illustrated in Fig. 2 showing an embodiment of a deflation valve assembly.
Fig. 8 is a cross-sectional view of the pump illustrated in Fig. 2 showing another embodiment of a cuff valve assembly.
Fig. 9 is a cross-sectional view of the pump illustrated in Fig. 3 in an inflation mode for inflation of the penile implant and the artificial urinary sphincter cuff.
Fig. 10 is a cross-sectional view of the pump illustrated in Fig. 3 in a deflation mode for deflation of the penile implant and the artificial urinary sphincter cuff.
Fig. 11 is a schematic view of one embodiment of the implantable system for treatment of climacturia illustrated in Fig. 1 implanted into a user.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

The term "proximal" as employed in this application means that part that is nearest the body of the user. The term "distal" as employed in this application means that part that is farthest from the body of the user. A distal end is the furthest endmost location of a distal portion of a thing being described, whereas a proximal end is the nearest endmost location of a proximal portion of the thing being described.

"Auto inflation" means an involuntary inflation of a cylinder implanted in a penis. Autoinflation occurs when the pressure of the liquid inside a reservoir that supplies the cylinder is increased, and the increased pressure forces liquid from the reservoir into the cylinder. The consequence is an unintended and undesirable erection of the penis. In one embodiment, the pump disclosed herein includes an anti-auto inflation (AAI) mechanism that is disposed in the pump body and has a seal that is biased to prevent liquid flow from bypassing the pump bulb and flowing directly from the reservoir to the cylinders.

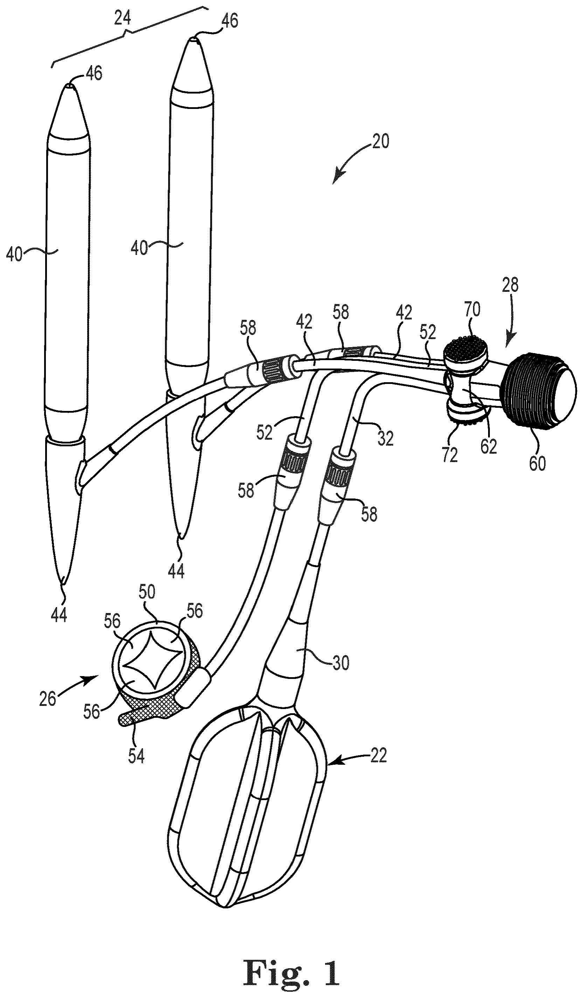

Fig. 1 is a perspective view of one embodiment of an implantable system 20 for treatment of climacturia. The system 20 includes a reservoir 22 configured to contain a liquid, a penile implant 24 that is implantable in a penis, a cuff 26 that is implantable around at least a portion of a urethra, and a pump 28 to move the liquid contained in the reservoir 22 into the penile implant 24 and into the cuff 26.

The reservoir 22 is sized to maintain a volume of liquid between about 50-300 ml and includes a neck 30 that is coupled with tubing 32 that connects the reservoir 22 with the pump 28. The reservoir 22 is suitable for implantation in the user either under the muscle layer(s) or subcutaneously between the skin and the muscle layers. In one embodiment, the reservoir 22 is provided as a "cloverleaf" style having multiple leaves that may be folded one against the other to compact the reservoir 22 for implantation through the pelvis and into the abdomen of the user. One suitable reservoir 22 is sized to retain approximately 130 mL of liquid and is available from Coloplast Corp., Minneapolis, MN.

The penile implant 24 includes at least one inflatable body that is implantable within the penis to form an erection when inflated with liquid. In one example, the penile implant 24 includes two inflatable bodies in the form of inflatable cylinders 40, where each of the inflatable cylinders 40 is implantable into a dilated corpus cavernosum of the penis. Each of the cylinders 40 is connected to the pump 28 by tubing 42 and includes a proximal end 44 opposite a distal end 46. During implantation, the proximal end 44 (also called a rear tip) is implanted toward the crus of the penis and the distal end 46 is implanted within the glans penis. The cylinders 40 are fabricated from material configured to collapse and be flexible when the cylinders 40 are deflated to provide the penis with a flaccid state. When collapsed, the cylinders 40 flatten and are floppy to provide the penis with a natural flaccid state. The cylinders 40 are inflatable with liquid to expand and become rigid to provide the penis with an erection. As a point of reference, the cylinders 40 are illustrated in an inflated state. Suitable material for fabricating the cylinders 40 includes silicone, biocompatible polymers such as urethanes, blends of polymers with urethane, copolymers of urethane, or the like. Suitable cylinders are available from Coloplast Corp., Minneapolis, MN.

The cuff 26 is implantable around at least a portion of a urethra and inflatable with the liquid contained in the reservoir 22. The cuff 26 has an inflatable belt 50 that is connected to the pump 28 with tubing 52. The inflatable belt 50 of the cuff 26 is, in one example, provided as an elongated strip 54 having a length that can be looped around the urethra and attached to itself. Since the inflatable belt 50 is looped around the generally cylindrical urethra, the inflatable belt 50 can include inflatable pillows 56 distributed along the interior length of the strip 54, where the inflatable pillows 56 expand to occupy the generally circular shape inside of the cuff 26 to better coapt the urethra when the pillows 56 are inflated. Alternatively, the inflatable belt 50 is ring-shaped (for example, like an annulus) and can be opened for placement around the urethra and closed to retain the inflatable belt 50 around the urethra. The cuff 26, when inflated, provides a coaptation of the urethra in treatment of climacturia.

Each of the sections of the tubing 32, 42, 52 can include a two-part connector 58 that allows the surgeon to select, or cut-to-size, a desired length for each of the tubings 32, 42, 52. When the surgeon has appropriately sized each of the tubings by cutting an excess portion off the tubings 32, 42, 52, then the two-part connector 58 is fitted to each opposing end of the cut tubings 32, 42, 52 and snap-fit together to bring the two parts of the tubings 32, 42, 52 together. Those familiar with such implantable connectors refer to the connector 58 by its tradename LUER-LOK small tubing connector.

The pump 28 receives liquid from the reservoir 22 through the tubing 32 and discharges the liquid to the penile implant 24 through the tubing 42 and to the cuff 26 through the tubing 52. The pump 28 includes a pump bulb 60 connected to a pump body 62, where the tubing 32, 42, and 52 connects to the pump body 62. The pressure for inflation of the penile implant 24 is generally in a range from about 10 pounds-per-square-inch (psi) to about 25 psi (a range between 69.0 kPa to 172.4 kPa), with a common inflation pressure for the penile implant being about 15 psi (103.4 kPa). The pressure for inflation of the cuff 26 is generally in a range from about 0.5 psi to 1.5 psi (3.5 kPa to 10.3 kPa). Thus, the pressure in the inflated cuff 26 is substantially lower than the pressure in the inflated penile implant 24 and the valves inside of the pump 28 are designed to accommodate this range of distinctly different pressure conditions. The inflated penile implant 24 and the inflated cuff 26 can be deflated by pressing surfaces 70, 72 on the pump body 62.

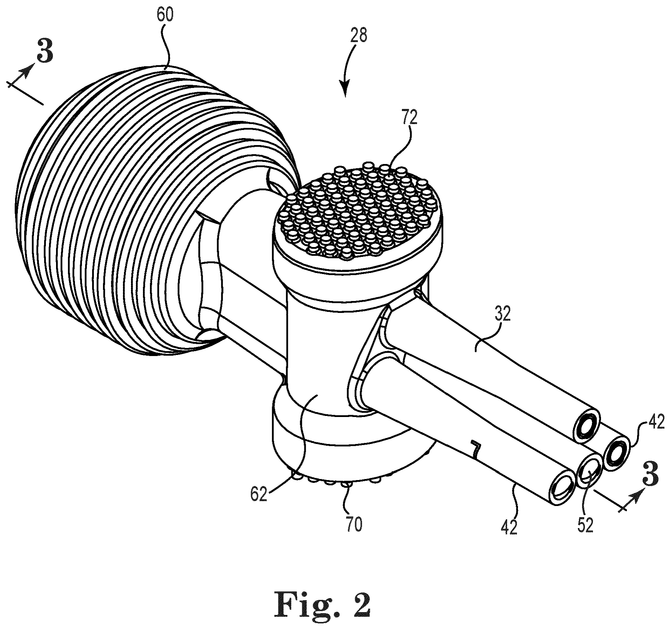

Fig. 2 is a perspective view of the pump 28. The pump bulb 60 is flexible and includes a ribbed accordion structure that allows the pump bulb 60 to collapse to drive liquid out of the pump bulb 60, through the pump body 62, and out of the tubes 42, 52. The pleated accordion structure is configured to recover to expand the bulb 60, which creates a negative local pressure in the bulb 60 that draws liquid out of the reservoir 22 (Fig. 1), through the tube 32 and the pump body 62, and into the pump bulb 60.

In one embodiment, the pump body 62 is integrally formed with the pump bulb 60 and includes a first activation surface 70 opposite a second activation surface 72. The activation surfaces 70, 72 (also called deflate pads) are illustrated as non-circular (elliptical) although other shapes for the activation surfaces 70, 72 are also acceptable. The pump body 62 encloses valves that direct the liquid to the penile implants 24 and the cuff 26 in additional to a deflate valve that operates to deflate the penile implant 24 and the cuff 26 when activated by pressing the activation surfaces 70, 72.

Generally, the pump 28 is implanted into the scrotum of the user, the cylinders 40 are implanted into the penis of the user, and the reservoir 22 is implanted within the abdomen of the user. The pump 28 is fabricated from material suitable for body implantation, such as silicone or the urethane-based materials described above for the cylinders 40 or the reservoir 22.

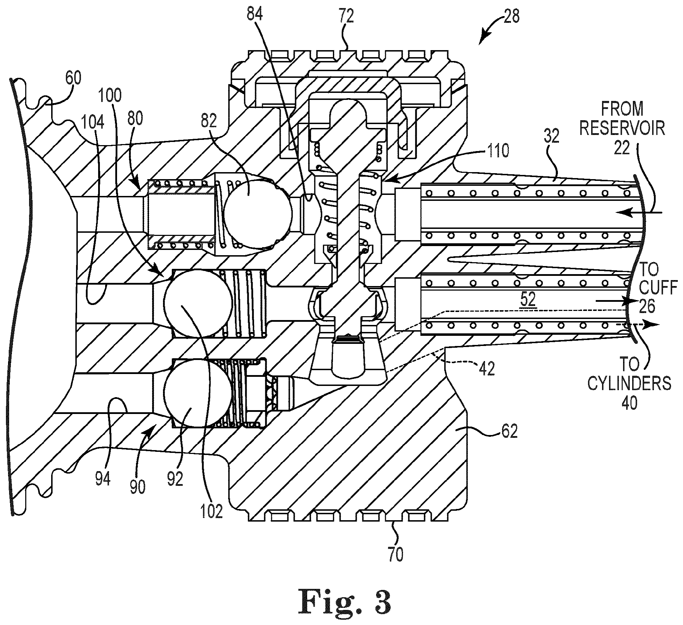

Fig. 3 is a cross-sectional view of the pump 28. The pump 28 includes an inlet valve assembly 80 having an inlet valve 82 disposed in an inlet channel 84 of the pump body 62, an exhaust valve assembly 90 having an exhaust valve 92 disposed in a first exhaust channel 94 of the pump body 62, a cuff valve assembly 100 having a cuff valve 102 disposed in a second exhaust channel 104 of the pump body 62, and a deflation valve assembly 110.

The inlet valve assembly 80 allows a portion of the liquid from the reservoir to enter the pump bulb 60 when the pump bulb is squeezed and released. The liquid from the reservoir 22 traverses through, or through and around, the deflation valve assembly 110 and then through the inlet channel 84 of the pump body 62 before entering the pump bulb 60.

The exhaust valve assembly 90 allows a first portion of the liquid drawn into the pump bulb 60 to flow to the penile implant cylinders 40 when the pump bulb is squeezed. The liquid in the pump bulb 60 flows through the first exhaust channel 94 and through, or through and around, the deflation valve assembly 110 before exiting through the tubing 42 to the cylinders 40. The exhaust valve assembly 90 is configured to inflate the cylinders to a use pressure in the range from about 10 - 25 psi (69.0 kPa to 172.4 kPa).

Likewise, the cuff valve assembly 100 allows a second and smaller portion of the liquid drawn into the pump bulb 60 to flow through the second exhaust channel 104 and through, or through and around, the deflation valve assembly 110 before exiting through the tubing 52 to the cuff 26. The cuff valve assembly 100 is configured to inflate the cuff 26 to a much lower use pressure in the range from about 0.5 psi to 1.5 psi (3.5 kPa to 10.3 kPa).

Thus, the valve assemblies 80, 90, 100 are useful to inflate both the cylinders 40 of the penile implant and the cuff 26 with the portion of the liquid drawn out of the reservoir 22. Repeatedly squeezing followed by recovery of the pump bulb 60 repeatedly draws a portion of the liquid out of the reservoir 22 and into the pump bulb 60 and displaces that portion of liquid into the penile implants 24 and the cuff 26. The user is instructed to repeatedly squeeze the pump bulb 60 until a desired level of rigidity (pressure) is achieved in the penile implants 24. The cuff valve assembly 100 is configured to ensure that the cuff 26 is not over inflated or over pressurized (the pressure is maintained at a level between 0.5 to 1.5 psi) even as the penile implant 24 is pressurized to the higher-pressure level between 10-25 psi. The deflation valve assembly 110 is operable to deflate the cylinders 40 and the cuff 26 by directing the liquid in the cylinders 40 and the cuff 26 back to the reservoir 22.

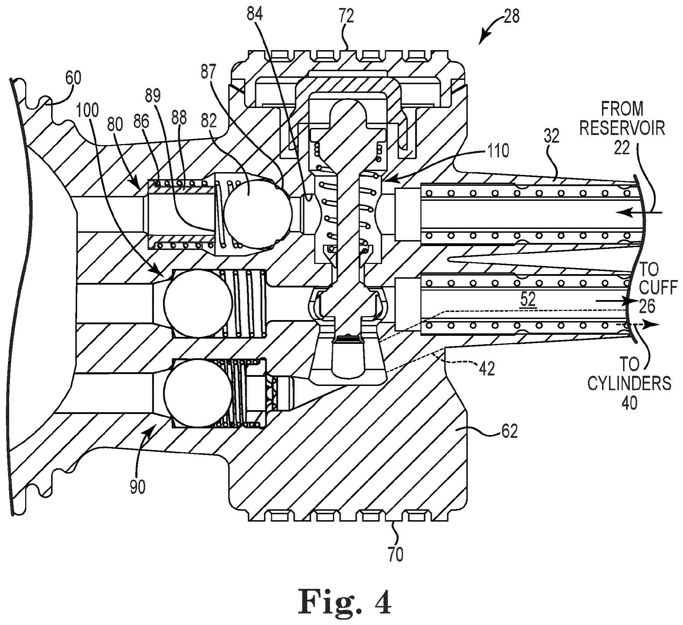

Fig. 4 is a cross-sectional view of the pump 28 showing the inlet valve assembly 80. The inlet valve assembly 80 provides a one-way valve allowing flow from the reservoir 22 into the bulb 60. In the case where the inlet valve 82 is a ball valve, a spring 86 is provided to bias the inlet ball valve 82 against a first valve seat 87. In some cases, the inlet channel 84 is reinforced or strengthened with a tubular sleeve 88 that is provided to resist a high-pressure flow (greater than about 25 psi) exiting the cylinders 40 during deflation and undesirably flowing past the inlet ball valve 82 and into the pump bulb 60. This situation is undesirable because the high-pressure backflow, when entering the pump bulb 60, can consequently and undesirably re-inflate the cylinders 40 during the deflation process. The tubular sleeve 88 provides a second valve seat 89 upstream of the valve 82 that receives the inlet ball valve 82 during such high-pressure spikes, which ensures that the high-pressure flow during deflation does not distort the pump body 62 and enter the pump bulb 60. In a typical use scenario, pressing the pump bulb 60 creates a low pressure zone, or suction, inside the pump bulb 60 that draws the liquid from the reservoir 22 into the pump bulb 60.

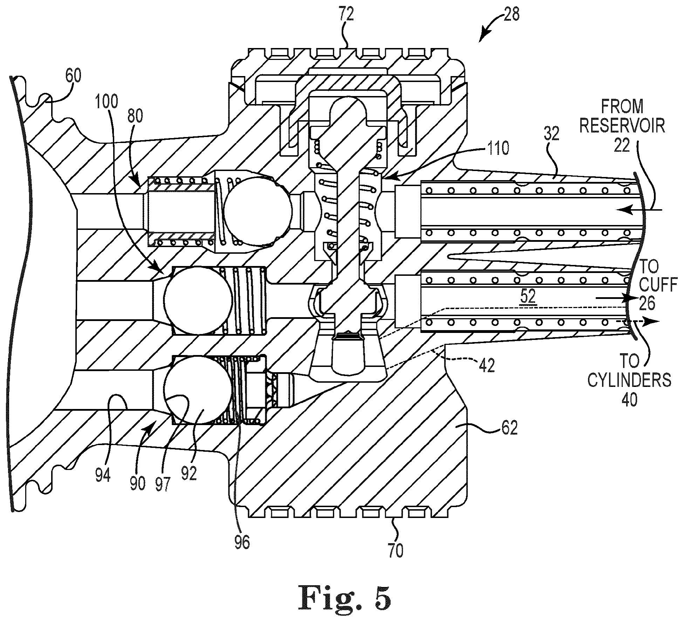

Fig. 5 is a cross-sectional view of the pump 28 showing the exhaust valve assembly 90. The exhaust valve assembly 90 provides a one-way valve allowing flow from the pump bulb 60 through the first exhaust channel 94 to the cylinders 40. In the case where the exhaust valve 92 is a ball valve, a spring 96 is provided to bias the exhaust ball valve 92 against a valve seat 97. In a typical use scenario, pressing the pump bulb 60 ejects a first portion of the liquid inside the pump bulb 60 through the first exhaust channel 94 to inflate the cylinders 40.

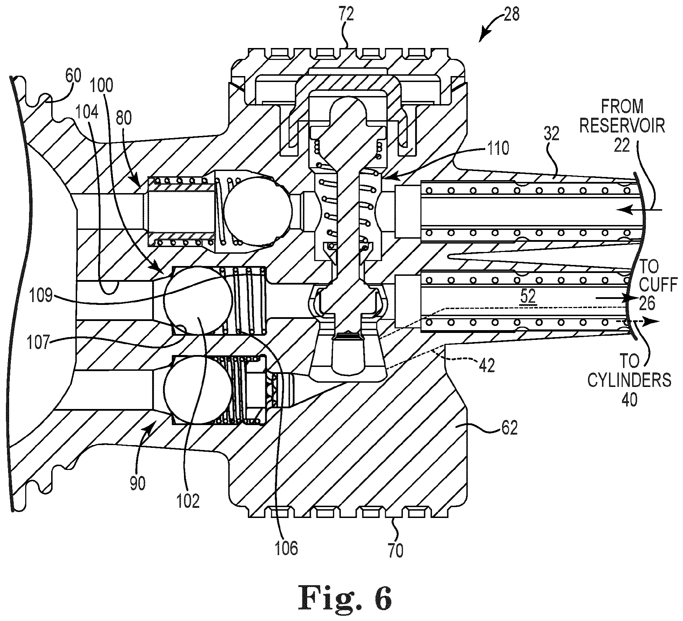

Fig. 6 is a cross-sectional view of the pump 28 showing the cuff valve assembly 100. The cuff valve assembly 100 provides a one-way valve allowing a second portion of the liquid from the pump bulb 60 to be ejected from the pump bulb 60 and flow through the second exhaust channel 104 to the cuff 26.

The cylinders 40 and the cuff 26 can be inflated simultaneously, but at widely different pressures. For example, the exhaust valve assembly 90 is configured to pressurize the penile implant cylinders 40 in a range from 5 pounds-per-square inch (psi) to 20 psi, which in cgs units is in a range from 34.5 kPa to 68.9 kPa and the cuff valve assembly 100 is configured to pressurize the cuff 26 in a range from 0.5 pounds-per-square inch (psi) to 1.5 psi, which in cgs units is in a range from 3.5 kPa to 10.3 kPa. In one embodiment, the cuff spring 106 has a spring constant selected to deflect to allow the cuff ball valve 102 to close the second exhaust channel 104 to prevent pressuring the cuff 26 above 1.5 psi (10.3 kPa).

In the case where the cuff valve 102 is a ball valve, a spring 106 is provided to bias the cuff ball valve 102 against a valve seat 107. The spring constant of the spring 106 is selected to allow the cuff ball valve 102 to move off the seat 107 for liquid pressures in a range from 0.5 psi to 1.5 psi, which in centimeter-gram-seconds units (cgs units) is in a range from 3.5 kPa to 10.3 kPa. In addition, the cuff valve assembly 100 is operable to close the second exhaust channel 104 of the pump body 62 to prevent pressure in the cuff 26 exceeding 3 psi, which in cgs units is 20.7 kPa. In a preferred embodiment, the cuff valve assembly 100 is configured to close the second exhaust channel 104 of the pump body 62 to prevent additional liquid exiting the pump bulb 62 and entering the cuff 16 when a pressure in the cuff is in a range from 0.5 pounds-per-square inch (psi) to 1.5 psi, which in cgs units is in a range from 3.5 kPa to 10.3 kPa. In this way, the cuff 26 that coapts the urethra will be pressurized in a range from 0.5 psi to 1.5 psi (3.5 kPa to 10.3 kPa), which is an effective range for closing the urethra and preventing the leakage of urine through the urethra. When the pressure in the flow of liquid leaving the pump bulb 60 and flowing through the second exhaust channel 104 of the pump body 62 exceeds the selected pressure, for example when the pressure in the flow exceeds 1.5 psi (10.3 kPa), the force of the flow applied to the ball valve 102 fully deflects the spring 106 to press the ball valve 102 against a second valve seat 109, thus effectively stopping the flow of the liquid into the cuff 26 and capping the pressure in the cuff 26 at about 1.5 psi (10.3 kPa). The spring constant of the spring 106 is selected to achieve a desired coaptation pressure in the cuff 26, and the spring constant of the spring 106 could fall in a range of values depending upon the diameter of the urethra and the amount of coaptation suited for closing the urethra.

As examples, the cuff 26 may be provided in various formats depending upon how much closure force, or coaptation, is desired to be applied to the urethra. A first alternative cuff 26 provides a first spring constant of the spring 106 is selected to allow the cuff ball valve 102 to move off the seat 107 for liquid pressures in a range from 0.5 psi to 0.7 psi (3.5 kPa to 4.8 kPa). A second alternative cuff 26 provides a second spring constant of the spring 106 is selected to allow the cuff ball valve 102 to move off the seat 107 for liquid pressures in a range from 0.7 psi to 0.85 psi (4.8 kPa to 5.9 kPa). A third alternative cuff 26 provides a third spring constant of the spring 106 is selected to allow the cuff ball valve 102 to move off the seat 107 for liquid pressures in a range from 0.85 psi to 1.0 psi (5.9 kPa to 6.9 kPa). A fourth alternative cuff 26 provides a fourth spring constant of the spring 106 is selected to allow the cuff ball valve 102 to move off the seat 107 for liquid pressures in a range from 1.0 psi to 1.1 psi (6.9 kPa to 7.6 kPa). A fifth alternative cuff 26 provides a fifth spring constant of the spring 106 is selected to allow the cuff ball valve 102 to move off the seat 107 for liquid pressures in a range from 1.1 psi to 1.3 psi (7.6 kPa to 9.0 kPa). Selecting the appropriate spring constant for the spring 106 allows the cuff 26 to be provided in a range of pressures suited to coapting the urethra to treat climacturia.

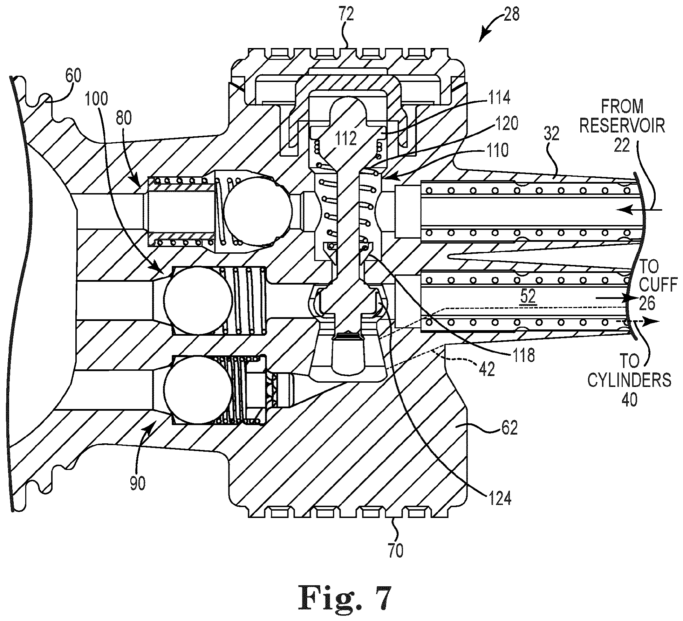

Fig. 7 is a cross-sectional view of the pump 28 showing the deflation valve assembly 110. The deflation valve assembly 110 is provided to deflate both the cylinders 40 and the cuff 26. The deflation valve assembly 110 includes a valve stem 112, a flange 114 disposed on a first end of the valve stem 112, a seal 118, a spring 120 that biases the seal 118 away from the flange 114, and a crown 124 attached to the valve stem 112 opposite the flange 114. In one embodiment, the spring 120 is a conical spring disposed between the flange 114 and the seal 96, with the end of the spring 106 against the flange 114 wider than an end of the spring 106 against the seal 118.

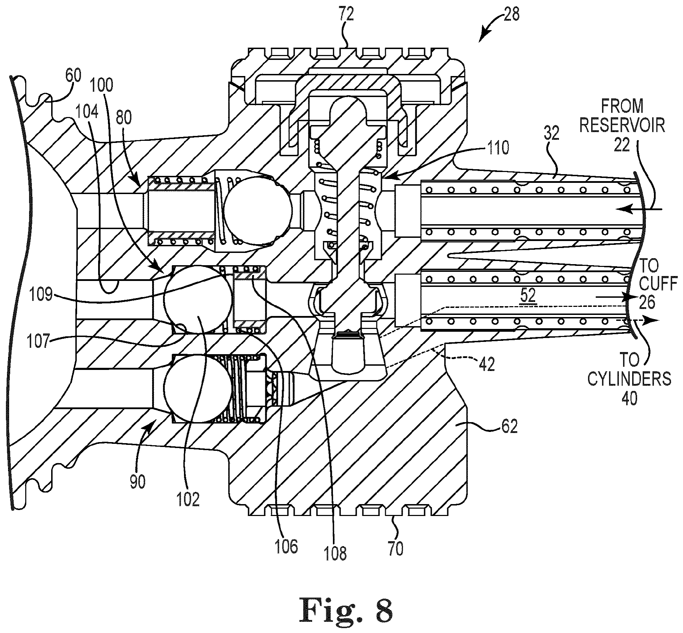

Fig. 8 is an alternative embodiment of the valve assembly 100 including a reinforcing sleeve 108 inserted within the second exhaust channel 104. The reinforcing sleeve 108 resists deformation of the pump body 62, and specifically deformation of the second exhaust channel 104, to resist over-pressurization of the cuff 26. It can be useful to provide the reinforcing tubular sleeve 108 having a second valve seat 109 downstream of the valve 102 to ensure sealing of the cuff ball valve 102, particularly as the pump bulb 60 is repeatedly squeezed to inflate the cylinders 40 to a pressure above 10 psi (68.9 kPa). When the inflation pressure of the liquid exiting the pump bulb exceeds 1.5 psi (10.3 kPa), the cuff ball valve 102 will seat against the second valve seat 109 and close off flow to the cuff 26, which prevents over pressurization of the cuff 26.

As described above, it is desirable to inflate the cuff 26 to a pressure in a range between 0.5 psi to about 1.5 psi (3.4 kPa to about 10.3 kPa). However, the pump bulb 60 is also employed to inflate the cylinders 40 to about 15 psi (103.4 kPa), and some users choose to inflate the cylinders 40 to a range above 15 psi (103.4 kPa), such as in a range between 15-25 psi (103.4 kPa - 172.4 kPa). The tubular sleeve 108 is provided to ensure that the high pressure delivered from the pump bulb 60 to the cylinders 40 does not undesirably skirt around the cuff ball valve 102 and over pressure the cuff 26. The tubular sleeve 108 provides the second valve seat 109 that receives the cuff ball valve 102 during the high-pressure spikes to ensure the cuff 26 remains pressurized in the range from 0.5 - 1.5 psi (3.4 kPa to about 10.3 kPa). In one embodiment, the tubular sleeve 108 is fabricated from stainless steel and includes the following exemplary dimensions: a wall with an inside diameter of about 0.095 inches (2.4 mm) and an outside diameter of about 0.130 inches (3.3 mm); the sleeve 108 has a length of about 0.165 inches (4.19 mm); the outside diameter of the flange portion of the sleeve 108 is about 0.156 inches (3.96 mm); and a thickness of the flange is about 0.015 inches (0.39 mm), although other dimensions are acceptable.

Suitable valves 80, 90, 100 include ball valves, poppet valves, and flexible disk valves such as a rubber flap style of valve.

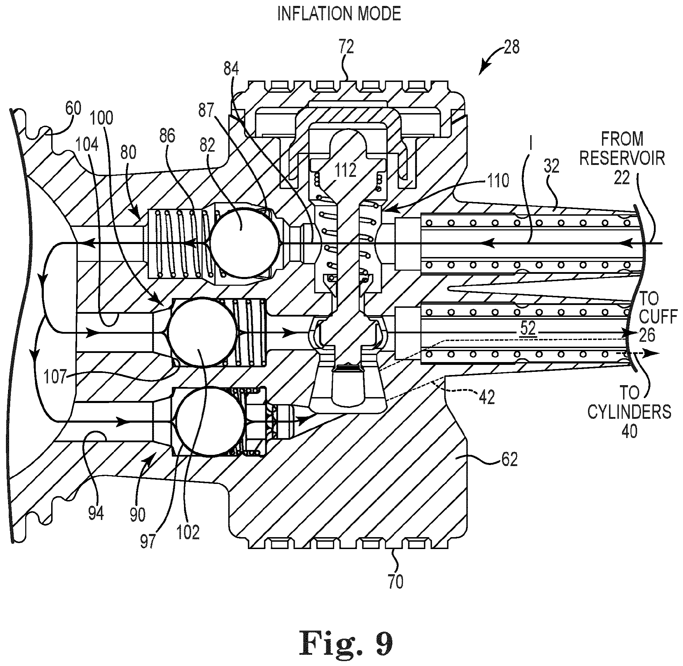

Fig. 9 is a cross-sectional view of the pump 28 of Figs. 3-7 in the inflation mode. When at rest, the inlet valve 82 is biased onto the valve seat 87 by the spring 86. When the pump bulb 60 is squeezed and expands to create a vacuum, liquid is drawn from the reservoir 22 and into the pump bulb 60. The liquid moves through the tubing 32, through inlet channel 84, and through the inlet valve assembly 80 along an inflation pathway I. The valve 82 is displaced away from the entry valve seat 87 to allow the liquid to flow around the valve 82, through the inlet valve assembly 80, and into the pump bulb 60.

When the pump bulb 60 is compressed, the liquid in the pump bulb 60 is ejected through the exhaust valve assembly 90 and through the cuff valve assembly 100 along the inflation pathway I, displacing the valve 92 from its seat 97 and displacing the valve 102 off its seat 107. The liquid is ejected from the pump bulb 60 and flows along the inflation pathway I through the exhaust valve assembly 90 and the cuff valve assembly 100 and around the deflation valve assembly 110, through the tubing 42 the cylinders 40 and through the tubing 52 to the cuff 26. The spring of the cuff valve assembly 100 is selected to allow the cuff to be pressurized in a range from 0.5 pounds-per-square inch (psi) to 1.5 psi, which in cgs units is in a range from 3.5 kPa to 10.3 kPa. Consequently, the cuff valve assembly 100 will "turn off" the pressurization to the cuff 26 as the exhaust valve assembly 90 continues to allow the cylinders 40 to be pressurized in a range from 5 pounds-per-square inch (psi) to 20 psi, which in cgs units is in a range from 34.5 kPa to 68.9 kPa.

In one embodiment, when the pump bulb 60 is squeezed, liquid moving through the exhaust valve assembly 90 forces the deflation valve 112 upward to seal the deflation valve 112 and prevent the liquid that is flowing toward the cylinders 40 from being diverted through the pump body 62 toward the reservoir 22. Subsequent multiple pumps of the pump bulb 60 transfers the liquid in the reservoir 22 through the pump body 62, to the pump bulb 60, and out of the pump bulb 60 to the cylinders 40 and to the cuff 26.

The deflation valve assembly 110 is placed within the pump body 62 transverse relative to the inflation pathway I. During inflation, both the inlet valve assembly 80 and the deflation valve assembly 110 close to prevent the liquid flowing from the pump bulb 60 to the cylinders 40 from undesirably being diverted back toward the reservoir 22. The deflation valve assembly 110 provides a onetouch release feature that opens the valve 112 for rapid deflation of the cylinders 40. For example, squeezing or pressing the deflations surfaces 70, 72 moves the valve 112 downward to allow the liquid in the cylinders 40 and the liquid in the cuff 26 to flow past the deflation valve assembly 110 and back to the reservoir 22.

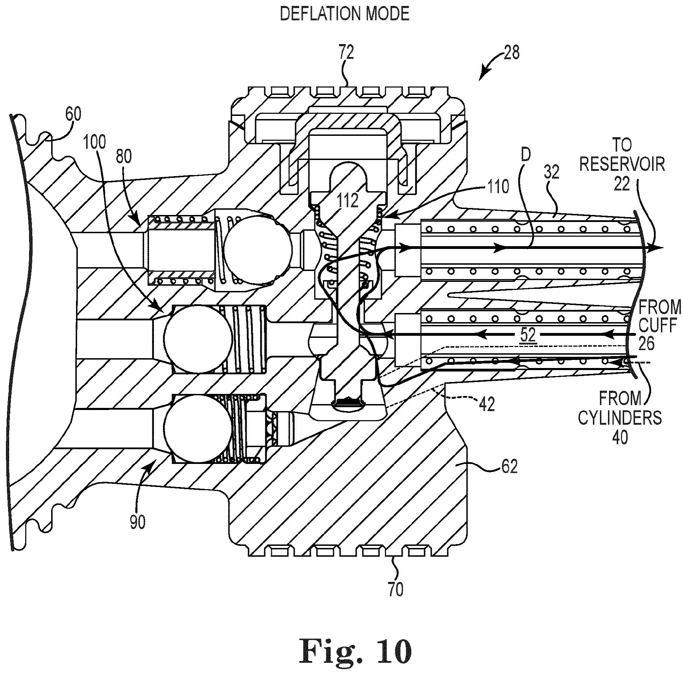

Fig. 10 is a cross-sectional view of the pump 28 of Figs. 3-7 in a deflation mode with the deflation valve assembly 110 displaced downward in the view to open a deflation flow path D from the cylinders 40 and the cuff 26 back to the reservoir 22. The deflation valve 112 allows for the rapid deflation of the cylinders 40 and the cuff 26 by providing the deflation pathway D that bypasses the pump bulb 60 and directs the liquid from the cylinders 40 and the cuff 26 along the deflation valve 112 and back to the reservoir 22. Pressing on the surfaces 70, 72 displaces the deflation valve 112 and opens the deflation flow path D. Since the cylinders 40 and the cuff 26 are pressurized, the liquid in the cylinders 40 and the cuff 26 will flow along the deflation path D and back to the generally lower pressure reservoir 22. The exhaust valve assembly 90 and the cuff valve assembly 100 block the flow of the liquid leaving the cylinders 40 and the cuff 26 from entering the pump bulb 60. This ensures that the liquid being forced from the cylinders 40 and the cuff 26 is diverted through the pump body 62 (away from the pump bulb 60) and back into the reservoir 22.

With reference to Fig. 4 and Fig. 10, the reinforcing sleeve 108 prevents the high-pressure liquid returning from the cylinders 40 from deforming portions of the pump body 62, which could result in the high-pressure liquid bypassing the inlet valve assembly 80, entering the pump bulb 60, and undesirably re-pressurizing the cylinders 40 during deflation. The reinforcing sleeve 108 thus provides an added level of reassurance to ensure that the cuff 26 is not over-pressurized.

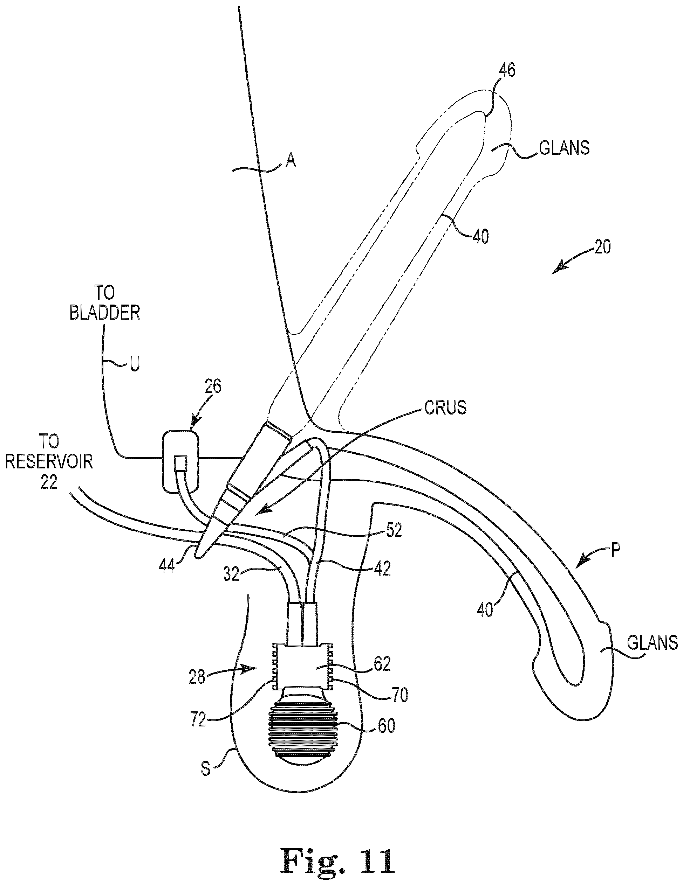

Fig. 11 is a schematic side view of the implantable system 20 for treatment of climacturia implanted in a user. The cylinders 40 are implanted in the penis P with the proximal end 44 inserted within the crus penis and the distal end 46 implanted within the glans penis. The pump 28 is implanted within the scrotum S and coupled to the reservoir 22 by tubing 32, coupled to the cylinders 40 by the tubing 42, and coupled to the cuff 26 by the tubing 52. The reservoir 22 is implanted within the abdomen A. Advantageously, the cuff 26 may also be implanted in the same procedure along with the implantation of the reservoir 22, the pump 28, and the cylinders 40.

The cuff 26 is implanted around a portion of the urethra U inferior the bladder. The system 20 is operable consistent with the description above to inflate the cylinders 40 with liquid from the reservoir 22 to provide the penis P with an erect state and to simultaneously inflate the cuff 26 to treat climacturia. The cylinders 40 and the cuff 26 can be deflated by activating (pressing) the surfaces 70, 72 to deflate the cylinders 40 and the cuff 26 to return the penis P to a flaccid state (as described relative to Fig. 10) and to open the cuff 26 for the passage of urine through the urethra.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of medical devices as discussed herein. Therefore, it is intended that this invention be limited only by the claims and the equivalents thereof.

## Claims

1. An implantable system comprising:
a reservoir configured to contain a liquid;
a penile implant that is implantable in a penis and inflatable with the liquid contained in the reservoir, where the penile implant, when inflated, is configured to provide the penis with an erection;
a cuff that is implantable around at least a portion of a urethra and inflatable with the liquid contained in the reservoir, where the cuff, when inflated, is configured to provide a coaptation of the urethra; and
a pump comprising a pump bulb connected to a pump body, the pump bulb operable to move the liquid contained in the reservoir into the penile implant and into the cuff, the pump comprising:
an inlet valve assembly comprising an inlet valve disposed in an inlet channel of the pump body and operable to allow a portion of the liquid contained in the reservoir to pass through the inlet channel and
into the pump bulb,
an exhaust valve assembly comprising an exhaust valve disposed in a first exhaust channel of the pump body and operable to allow a first portion of the liquid in the pump bulb to pass through the first exhaust channel and into the penile implant,
a cuff valve assembly comprising a cuff valve disposed in a second exhaust channel of the pump body and operable to allow a second portion of the liquid in the pump bulb to pass through the second
exhaust channel and into the cuff;
wherein operation of the pump bulb displaces the first portion of the liquid and the second portion of the liquid out of the pump bulb to inflate both the penile implant and the cuff.

2. The implantable system of claim 1, wherein operation of the pump bulb simultaneously inflates both the penile implant and the cuff.

3. The implantable system of claim 1 or 2, wherein the exhaust valve comprises a ball valve and the exhaust valve assembly further comprises a spring to bias the ball valve, and the exhaust valve assembly is configured to pressurize the penile implant in a range from 34.5 kPa to 68.9 kPa (5 pounds-per-square inch (psi) to 20 psi).

4. The implantable system of any one of claims 1-3, wherein the cuff valve comprises a cuff ball valve and the cuff valve assembly further comprises a spring to bias the cuff ball valve, and the cuff valve assembly is configured to pressurize the cuff in a range from 3.5 kPa to 10.3 kPa (0.5 pounds-per-square inch (psi) to 1.5 psi).

5. The implantable system of claim 1, wherein the exhaust valve assembly further comprises an exhaust spring biasing an exhaust ball valve onto an exhaust valve seat, and the cuff valve assembly further comprises a cuff spring biasing a cuff ball valve onto a cuff valve seat;
wherein the exhaust valve assembly is configured to pressurize the penile implant in a range from 34.5 kPa to 68.9 kPa (5 pounds-per-square inch (psi) to 20 psi);
wherein the cuff spring has a spring constant selected to deflect to allow the cuff ball valve to close the second exhaust channel to prevent pressuring the cuff above 10.3 kPa (1.5 psi).

6. The implantable system of claim 1, wherein operation of the pump bulb simultaneously inflates both the penile implant and the cuff, and the exhaust valve assembly is operable to pressurize the penile implant in a range from 34.5 kPa to 68.9 kPa (5 pounds-per-square inch (psi) to 20 psi), and the cuff valve assembly is operable to close the second exhaust channel of the pump body to prevent pressure in the cuff exceeding 20.7 kPa (3 psi).

7. The implantable system of any one of claims 1-6, wherein the cuff valve assembly is configured to close the second exhaust channel of the pump body to prevent additional liquid exiting the pump bulb and entering the cuff when a pressure in the cuff is in a range from 3.5 kPa to 10.3 kPa (0.5 pounds-per-square inch (psi) to 1.5 psi).

8. The implantable system of any one of claims 1-7, wherein the second exhaust channel of the pump body comprises a reinforcing sleeve, and the reinforcing sleeve provides a valve seat downstream of the cuff valve.

9. The implantable system of any one of claims 1-8, further comprising:
a deflation valve disposed in the pump body, with the second exhaust channel of the cuff valve assembly located between the pump bulb and the deflation valve;
wherein the deflation valve has an open deflate position that allows pressurized liquid in the penile implant and pressurized liquid in the cuff to flow back into the reservoir.

10. The implantable system of claim 9, wherein the deflation valve is disposed in the pump body transverse to the inlet valve assembly and the exhaust valve assembly.

11. The implantable system of claim 10, wherein the deflation valve is movable linearly and squeezing the pump body displaces the deflation valve transversely to the open deflate position to allow the pressurized liquid in the penile implant and pressurized liquid in the cuff to bypass the pump bulb and flow back into the reservoir.
